Europäisches Patentamt

European Patent Office

Office européen des brevets

(19)

(11) Veröffentlichungsnummer: **0 231 816 B1**

# (12) EUROPÄISCHE PATENTSCHRIFT

(45) Veröffentlichungstag der Patentschrift: 29.05.91

(21) Anmeldenummer: 87100792.8

(22) Anmeldetag: 21.01.87

(51) Int. Cl.⁵: **A61K 37/66**, A61K 47/38, A61K 47/42

Die Akte enthält technische Angaben, die nach dem Eingang der Anmeldung eingereicht wurden und die nicht in dieser Patentschrift enthalten sind.

(54) **Pharmazeutische Zubereitungsformen zur Stabilisierung von Interferon alpha.**

(30) Priorität: 05.02.86 DE 3603444

(43) Veröffentlichungstag der Anmeldung:
12.08.87 Patentblatt 87/33

(45) Bekanntmachung des Hinweises auf die Patenterteilung:
29.05.91 Patentblatt 91/22

(84) Benannte Vertragsstaaten:
AT BE CH DE ES FR GB GR IT LI LU NL SE

(56) Entgegenhaltungen:
EP-A- 0 139 286
EP-A- 0 152 345
EP-A- 0 159 168
EP-A- 0 162 332

(73) Patentinhaber: **Dr. Karl Thomae GmbH**
**Postfach 1755**
**W-7950 Biberach (Riss)(DE)**

(72) Erfinder: **Franz, Helmut, Dr.**
**Obere Au 2**
**W-7950 Biberach 1(DE)**

**Beschreibung**

Abhängig von ihren biologischen und chemischen Eigenschaften und ihrem Syntheseort werden Human-Interferone und Interferone allgemein in drei Gruppen unterteilt, die mit Interferon alpha, Interferon beta und Interferon gamma bezeichnet werden entsprechend IFN alpha, IFN beta, IFN gamma. Der Syntheseort von IFN alpha, auf das sich die vorliegende Erfindung bezieht, sind die Leucozyten, die nach Stimulation oder Induktion durch entsprechende Induktoren (z. B. Viren oder poly I poly C)) IFN alpha synthetisieren und freisetzen. Mit geeigneten DNA-Sequenzen transformierte Bakterien können IFN alpha synthetisieren, die eine dem genuinen Human IFN alpha analoge biologische Aktivität besitzen, mit dem Vorteil, daß hochgereinigte und im Wesentlichen von Kontaminationen freie Proteine in vergleichsweise großen Mengen erhalten werden können, die für die klinische Anwendung, z.B. gegen verschiedene Virusinfektionen und Malignome, einsetzbar sind.

Alle bekannten Interferone wirken antiviral, antiproliferativ und - jedoch verschieden stark - immunmodulierend. Anfänglich als antivirales Agens erkannt, wird heute ihr therapeutischer Nutzen auch als antineoplastisches Mittel gesehen.

Hinsichtlich Wirkungsqualität und Nebenwirkungsrate kommt der Applikationsroute eine besondere Bedeutung zu. Gerade bei der systemischen Anwendung von Interferonen imponieren die Nachteile, da sie nicht nur das Zielgewebe, sondern auch gesunde, nicht infizierte oder nicht transformierte Zellen oder Organe erreichen und beeinflußen und daher Nebenwirkungen erzeugen, die einem intravenösen oder intramuskulären Einsatz entgegenstehen können. Außerdem zeigte die systemische Applikation nicht immer die erwünschte Effektivität. In einzelnen Fällen mag eine direkte Injektion zwar möglich sein, das Mittel der Wahl, z. B. bei Virus - oder tumorbedingten malignen oder anderen mit Interferon therapierbaren Erkrankungen, die einer äußerlichen und lokalen Behandlung zugänglich sind (z. B. Haut, Augen, Nase), ist aber eher die topische Anwendung von Interferon bzw von IFN alpha, auf das sich diese Erfindung bezieht. In jüngster Zeit wurde von verschiedenen Seiten über die topische Applikation von Human-Leucocyten-Interferon, synonym mit IFN alpha, berichtet, wobei überraschenderweise eine hohe Erfolgsquote bei verschiedenen malignen Erkrankungen festgestellt werden konnte (z. B. Ikic et al, Lancet, 1022-24, 1981, Vol.1 ; Ikic et al., Lancet 1025-27, 1981, Vol.1). Es ist abzusehen, daß der topischen Anwendung von Interferon alpha zur Behandlung verschiedener viraler oder tumorbedingter Erkrankungen in Zukunft ein zunehmender medizinischer Stellenwert zukommen wird.

Mit der topischen Anwendung von IFN alpha als auch allgemein von Interferonen sind unter verschiedenen Gesichtspunkten erhebliche Schwierigkeiten verbunden. Unter dem Gesichtspunkt der pharmazeutischen Formulierung ist zu fordern, daß das biologisch aktive IFN alpha eine genügend hohe Stabilität erhält, um über eine ausreichend lange Zeit sowohl bei Raumtemperatur als auch bei Körpertemperatur zu wirken bzw. über einen geeigneten Zeitraum von mehreren Stunden "in situ" wirksam zu bleiben. Bekannt ist, daß IFN alpha ein sehr temperaturinstabiles Protein ist. Erhebliche Aktivitätsverluste von bis zu 80% wurden innerhalb von wenigen Wochen bei 4° C bei einem Human-Leucocyten-Interferon-haltigen Gel beobachtet, das als Gelkomponente Carboxymethylcellulose enthielt (Moller B.R. et al., Obstetrics & Gynecology 62, 625-629, 1983) und für die topische Anwendung zur Behandlung des Carcinoma in situ der Cervix eingesetzt wurde.

Die daher notwendigen stabilitätserhaltenden Komponenten müssen so beschaffen sein, daß sie weder Unverträglichkeitsreaktionen am Ort der Applikation verursachen, denkbar durch Wirkung von biologisch aktiven Fremdsubstanzen, noch die Penetration von IFN alpha beeinträchtigen oder die Diffusion verhindern.

In der letzten Zeit wurden verschiedene Komponenten zur Stabilisierung von Interferon benutzt, wobei Estis, Evans und Testa Hydroxyäthylcellulose als Trägermaterial zur Herstellung von Interferon-haltigen Gelen oder Salben eingesetzt haben, aber erhebliche Aktivitätsverluste von IFN alpha, IFN beta oder IFN gamma unter anwendungsrealistischen Bedingungen nur durch Zusatz eines Proteaseinhibitors verhindern konnten (EP-A-0 152 345).

Dem Fachmann ist bekannt, daß mit Hydroxyäthylcellulose ein zur Herstellung von Gelen dieser oder auch anderer Art bestgeeignetes Mittel, z. B. aufgrund seiner Hitzestabilität, vorliegt und kommerziell erhältlich ist.

Der besondere Vorteil von Hydroxyäthylcellulose zur Herstellung einer IFN alpha-haltigen Matrix liegt gegenüber anderen Trägern darin, daß sie bei Siedetemperatur von Wasser nicht koaguliert bzw. geliert, gut wasserlöslich ist, und daß die Viskosität einer solchen Lösung mit abnehmender Temperatur zunimmt.

Eine Stabilisierung von Interferon bzw. von IFN alpha durch Hydroxyäthylcellulose allein und unter physiologischen Temperaturen ist nicht zu erreichen. Auch ist dem Fachmann bekannt, daß nicht hochgereinigte und daher kontaminierte Proteinpräparate - wie z.B. Interferone - unter geeigneten Bedingungen der

2

Hydrolyse durch Proteasen ausgesetzt sind. Andere Versuche, in pharmazeutischen Präparaten enthaltene Interferone eine geeignete Stabilität zu verleihen, wurden von Akagi, Miura und Hoshino (EP-A-0 150 067) mit von Hydroxyäthylcellulose chemisch unterschiedlichen Polysacchariden, wie Dextran und Hydroxyäthylstärke unter Zusatz von Human Serum Albumin, von Mono- oder Disacchariden oder von Aminosäuren durchgeführt, wobei Präparate vornehmlich für die parenterale, systemische Anwendungsroute via intravenöser oder intramuskulärer Injektion beschrieben wurden. Darüber hinaus wurde von Terano nur allein auf Basis einer chemisch modifizierten Gelatine, ebenfalls für ein zu injizierendes pharmazeutisches Interferon-Präparat, eine Stabilisierung von Interferon in wässriger Lösung beschrieben, um damit Human Serum Albumin als bekanntes Interferon-stabilisierendes Mittel für die Injektion zu substituieren (EP-A-0 162 332).

Es ist dem Fachmann seit langem bekannt, daß Gelatine per se einen auf Interferone stabilisierenden Effekt aufweist (Sedmak J.J. and Grossberg, S.E. Tex. Rep. Biol. Med. 41, 274-279, 1982), und daß Gelatine und chemisch modifizierte Derivate davon auf einige andere biologische Proteine einen gewissen stabilisierenden Effekt ausüben (z.B. DE-A-1 118 732, EP-A-0 156 242).

Für die Stabilisierung von Interferon in Gelen, Salben, Suppositorien, Sprays usw. zur topischen Anwendung sind auch verschiedene Zuckeralkohole wie Glycerol, Erythoritol, Arabitol, Xylitol, Sorbitol und Mannitol sowie verschiedene Zuckersäuren wie Iduronsäure, Galacturonsäure und andere Uronsäuren,Ascorbinsäure und deren Salze als auch verschiedene organische Puffersysteme wie Citratpuffer, Succinatpuffer, Tartratpufer, Fumaratpuffer, Glyconatpuffer, Oxalatpuffer, Lactatpuffer und Acetatpuffer offenbart worden, in Verbindung mit verschiedenen pharmazeutischen Trägerstoffen wie Wachse, Natriumcarboxymethylcellulose, Carboxyvinylderivate und Wasser (EP-A-0 080 879).

Diese letztgenannten als auch die vorhergenannten Interferon-stabilisierenden Komponenten sowie vor allem die Zusammensetzungen der pharmazeutischen Formulierungen und deren Anwendungsmodalitäten (z.B. EP-A-0 162 332) sind grundsätzlich verschieden von der vorliegenden Erfindung und mit dieser nicht vergleichbar.

Die Aufgabe der vorliegenden Erfindung war es, eine Trägermatrix für hochgereinigtes Interferon alpha, speziell für rekombinant hergestelltes IFN alpha zu finden, die mindestens den obig aufgezählten Anforderungen entsprechen und zum anderen eine sowohl unter herstellungstechnischen als auch unter ökonomischen Gesichtspunkten so günstig wie mögliche, d.h. einfache Zusammensetzung, aufweisen sollte, ohne eine Einbuße einer geforderten optimalen Wirksamkeit des in der Trägermatrix enthaltenen biologisch aktiven Wirkstoffes IFN alpha zur topischen Anwendung zu bewirken. Durch die vorliegende Erfindung wurde dieser Aufgabenkomplex dadurch gelöst, daß Hydroxyäthylcellulose oder Hydroxypropylmethylcellulose oder Mischungen davon und Gelatine, saver oder alkalisch aufgeschlossen, miteinander vereint wurden und somit ein Träger und Stabilisator für IFN alpha geschaffen werden konnte, der die oben beschriebenen Anforderungen nicht nur erfüllt, sondern überraschenderweise dem IFN alpha eine besonders stabile biologische Aktivität verleiht und somit bestens für die topische Anwendung von hochgereinigtem insbesondere über rekombinante DNA-Technologie hergestelltem IFN alpha geeignet ist.

Ein weiterer Vorteil dieser Erfindung besteht darin, daß der gefundene Träger/Stabilisator-Komplex frei von Human Serum Albumin ist, das bekannterweise und insbesondere bei der topischen Anwendung gerade über längere Zeiträume zu lokalen Immunreaktionen bis zu Allergosen führen kann. Die vorliegende Erfindung bezieht sich daher auf eine verbesserte pharmazeutische Formulierung für die topische Anwendung von IFN alpha. Erfindungsgemäß wurde dabei festgestellt, daß beide als Träger und Stabilisator fungierenden Einzelkomponenten zusammen, nämlich Hydroxyäthylcellulose oder Hydroxypropylmethylcellulose oder Mischungen davon und Gelatine, eine über das zu erwartende Maß hinausgehende aktivitätserhaltende Wirkung auf IFN alpha ausüben, wobei gerade Berücksichtigung finden muß, daß IFN alpha und Interferone allgemein dafür bekannt sind, z.B. gegenüber physikalischen Einflüssen, instabile Proteine zu sein - sowohl in "nackter" Form als auch inkorporiert in anderen Gelmatrizes (z. B. Moller B.R. et al., siehe oben), so daß den erfindungsgemäßen Zubereitungsformen überraschend und in unvorhersehbarer Weise ein synergistischer Effekt hinsichtlich der Stabilistätserhöhung von IFN alpha eigen bzw. inhärent ist. Somit dient die Gelatine in der vorliegenden Erfindung nicht für sich allein als stabilisierendes Mittel, sondern entfaltet erst im Komplex zusammen mit Hydroxyäthylcellulose den festgestellten und erstaunlichen synergistischen Stabilisierungseffekt für IFN alpha, auf den sich diese Erfindung bezieht.

Die erfindungsgemäßen, topisch zu applizierenden pharmazeutischen Zubereitungsformen für IFN alpha werden vorzugsweise als Virusstatika angewendet, wobei der therapeutische Einsatz im dermalen, nasalen, ophthalmischen und intravaginalen Bereich, z.B. bei Herpes labialis, Herpes genitalis inclusive Papilloma-Infektionen, Warzen und bei Herpes Zoster-Infektionen, erfolgt. Der Anwendungsumfang umfaßt auch die topische Behandlung von Paracancerosen und Cancerosen.

Gegenstand dieser Erfindung sind topisch anzuwendende pharmazeutische Zubereitungsformen mit einer Stabilisierung des darin enthaltenen Wirkstoffs Human Interferon alpha (IFN alpha), bestehend aus 1.

3

einem Träger und Stabilisator im Komplex für den Wirkstoff, 2. einem Antiadhäsionsmittel und 3. gegebenfalls sonstigen üblichen Wirkstoffen wie Puffer und Salze, Feuchthalter, Konservierungsstoffe, Penetrationsförderer, Vehikel.

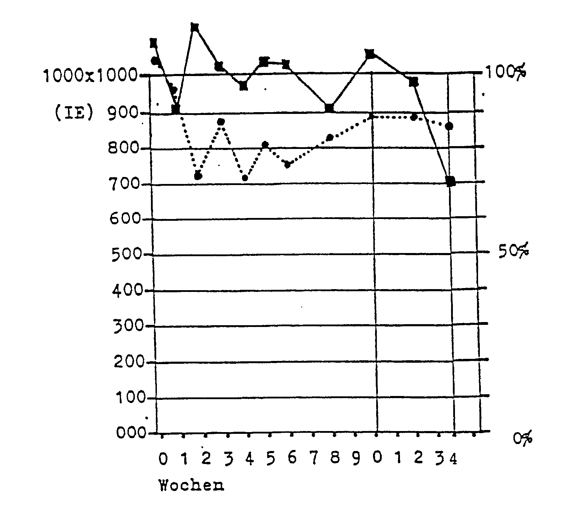

Als Träger und Stabilisator für IFN alpha werden Hydroxyethylcellulose, Hydroxypropylmethylcellulose oder Mischungen davon zusammen mit Gelatine, sauer oder alkalisch aufgeschlossen, eingesetzt. Als bevorzugter Träger und Stabilisator für IFN alpha wird Hydroxyäthylcellulose mit einem Substitutionsgrad von 2,5 in bestimmten Mischungsverhältnissen von jeweils 0,1 bis 2 Gew.% und Gelatine von 0,1 bis 10 Gew.%, jeweils bezogen auf das fertige Produkt, benutzt. Damit wird eine besondere Stabilisierung speziell von IFN alpha erreicht, wie es auch die durchgeführten Versuche über das Stabilitätsverhalten beispielhaft belegen und wie es die in Abb. 1 bis 3 gezeigten Kurvenverläufe, die einen Versuchszeitraum von 14 Wochen bei Lagertemparaturen jeweils von -10, +4 und +21° C umfassen, beispielhaft zeigen.

Als Antiadhäsionsmittel zur besseren Spreitung der Gele und als antiadhäsive Filtrationshilfe werden anionaktive, kationaktive sowie neutrale oberflächenaktive Substanzen benutzt, vorzugsweise werden polyäthoxylierte Sorbitan-Ester wie Polysorbat 20, 60, 80 benutzt.Als anorganische und organische Pufferlösungen haben sich mit einem für IFN alpha - Präparationen optimalen pH im Bereich von 6,0 bis 7,4 Natriumphosphat-Puffer, Kaliumhydrogenphosphat-Natriumdihydrogenphosphat-Puffer sowie Zitronensäurephosphat-Puffer, Kalium-Natrium-glutamat-phosphat-Puffer und Succinat-Puffer bewährt.

Für die erfindungsgemäßen IFN alpha haltigen Zubereitungsformen eignen sich Feuchthaltesubstanzen wie Glycerin, 1,2-Propylenglykol, Sorbit, Butylengklykol sowie Polyole (z. B. Polyethylenglykol 400).

Geeignete hautverträgliche Konservierungsstoffe sind z. B. p-Hydroxybenzoesäureester (Nipa-Ester), vorzugsweise p-Hydroxybenzoesäuremethylester; des weiteren Benzoesäure, Sorbinsäure, Chlorhexidindigluconat, Benzalkoniumchlorid und Hexadecyltrimethylammoniumbromid (Cetrimoniumbromid).

Ein geeigneter mit IFN alpha 2 kompatibler Penetrationsförderer ist bis zu 50 Gew.% Dimethylsulfoxid (DMSO).

Als Vehikel ist bei nichtalkoholischen Hydrogelen Wasser bestens geeignet.

Geeignete pharmazeutische Formen für die topische Anwendung auf der Haut, im Auge oder in der Nase oder sonstigen Körperhöhlen sind z. B. Hydrogele. Diese enthalten im allgemeinen 0,1 bis 2,0 Gew.% sowohl an Gelatine als auch an den Cellulosederivaten, wobei die Verhältnisse je nach gewünschter Viskosität aufeinander abgestimmt werden müssen. Im Falle der Fertigung von Vaginalkugeln ist der Anteil an Gelatine bis auf 10 Gew.% zu erhöhen, ebenfalls in Abhängigkeit von der gewünschten Viskosität.

Die Herstellung eines Hydrogels erfolgt im allgemeinen in der Weise, daß Gelatine in einer auf das fertige Produkt bezogenen Menge von 0,1 bis 2,0 Gew.-% bei Temperaturen bis zu 80° C in Wasser gelöst wird, dann die übrigen Hilfsstoffe, wie Feuchthalter, Salze, Puffersysteme und Penetrationsförderer und anschließend die Hydroxyethylcellulose, die Hydroxypropylmethylcellulose oder ein Gemisch hiervon, in die Lösung eingebracht werden. In die auf Raumtemperatur abgekühlte Lösung trägt man schließlich das Interferon alpha in einer Menge von $1 \times 10^5$ bis $1 \times 10^{10}$ IE/100 g fertiges Produkt ein und sorgt für eine homogene Verteilung. Das fertige Produkt wird steril abgefüllt.

Zur Herstellung von Zubereitungsformen für die intravaginale Applikation wird Hydroxyethylcellulose in einer Menge von 0,1 bis 2,0 Gew.-% zusammen mit Gelatine in einer Menge bis zu 10 Gew.-% bei Temperaturen bis zu 60° C in einem Wasser-Glycezin-Gemisch gelöst, bei einer Temperatur von 40 bis 50° C eine Polysorbat-haltige salzsaure IFN alpha-Lösung vorsichtig eingerührt und diese Lösung sofort in geeignete Formen gegossen.

In den folgenden Beispielen wird die Herstellung von topisch anzuwendenden pharmazeutischen Zubereitungsformen zur Stabilisierung des darin enthaltenen IFN alpha im Detail beschrieben, die sich gemäß der vorliegenden Erfindung in der Hauptsache aus Celluloseerivaten und Gelatine, Antiadhäsionsmitteln sowie dem darin enthaltenen Wirkstoff IFN alpha und verschiedenen Zusatzstoffen zusammensetzen.

In den Beispielen bedeuten:

| | |
|---|---|
| Nipagin® M | = p-Hydroxybenzoesäuremethylester, |
| Polysorbat 20 | = Polyoxyethylen(20)sorbitanmonolaurat (Tween[R]20); |
| Polysorbat 60 | = Polyoxyethylen(20)sorbitanmonostearat (Tween[R]60), |
| Polysorbat 80 | = Polyoxyethylen(20)sortibanmonooleat (Tween[R]80), |
| Benzalkoniumchlorid | = Mischung aus Alkyldimethylbenzylammoniumchloriden mit Alkylgruppen von 8 bis 18 Kohlenstoffatomen, |
| Cetrimoniumbromid | = Hexadecyltrimethylammoniumbromid, |

$3,2 \times 10^8$ I.E. Interferon-alpha-2 entsprechen 1 mg dieser Substanz.

Beispiel 1 (Hydrogel)

4

In 100 g Gelsubstanz sind enthalten :

| | |
|---|---|
| IFN alpha 2 | $1 \times 10^8$ IE |
| Nipagin M | 0,20 g |
| Gelatine, Bloom 160-200 | 0,50 g |
| NaCl | 0,48 g |
| KCL | 0,012 g |
| $NaH_2PO_4 \times 2 H_2O$ | 0,0593 g |
| $K_2HPO_4 \times 3 H_2O$ | 0,0407 g |
| Glutaminsäure-Na $\times$ $H_2O$ | 0,0636 g |
| Polysorbat 20 | 0,1 g |
| Hydroxyäthylcellulose (z.B. Natrosol$^R$ 250 HX) | 1,75 g |
| Wasser, deionisiert ad 100 g | 96,7944 g |

Die Präparation des IFN alpha 2 - Hydrogels erfolgt dadurch, daß Nipagin M in 80° C heißem Wasser gelöst und die Lösung anschließend auf Raumtemperatur (RT) abgekühlt wird, wobei während der Abkühlphase die Gelatine in diese Lösung eingebracht wird. Nach der vollständigen Lösung der Gelatine werden bei RT konsekutiv NaCl, KCL, NaH₂PO₄ x 2H₂O, K₂HPO₄ x 3 H₂O, Glutaminsäure-Na x H₂O sowie Polysorbat 20 zu dieser Lösung dazugegeben. Nach anschließender Sterilfiltration wird mikrobiologisch reine Hydroxyäthylcellulose eingestreut und die Lösung unter Rühren wieder auf 80° C erwärmt. Nach dem Abkühlen auf RT wird unter vorsichtigem Rühren die salzsaure, 1 % Polysorbat-haltige IFN alpha 2 - Lösung, bestehend aus einem in 0.01 N HCl gelöstem IFN alpha 2 - Lyophilisat, dazugegeben und homogen verteilt. Die Abfüllung dieses Gemisches erfolgt unter laminaren Air-flow-Bedingungen in sterile Aluminium-Tuben.

Beispiel 2 (Hydrogel)

In 100 g Gelsubstanz sind enthalten :

In 100 g Gelsubstanz sind enthalten :

| | |
|---|---|
| - IFN-alpha-2 | $1 \times 10^{10}$ I. E. |
| - Hydroxyäthylcellulose (z. B. Natrosol$^R$ 250 HX) | 2, 0 g |
| - Gelatine, Bloom 160 - 200 | 0, 1 g |
| - NaCl | 0, 35 g |
| - $Na_2HPO_4 \times 2H_2O$ | 0, 16 g |
| - $KH_2PO_4$ | 0, 09 g |
| - 1, 2-Propylenglykol | 10, 00 g |
| - Polysorbat 80 | 0, 1 g |
| - Benzalkoniumchlorid 90 % | 0, 02 g |
| - Wasser deion. | ad 100 g |

Die Herstellung des IFN-alpha 2-Hydrogels erfolgt dadurch, daß Benzalkoniumchlorid bei 30° C in dem Wasser gelöst wird, anschließend wird bei RT das Propylenglykol zugesetzt und die Gelatine in die Lösung eingebracht. Nach vollständiger Lösung der Gelatine werden bei RT nacheinander NaCl, $Na_2HPO_4 \times 2H_2O$, $KH_2PO_4$ sowie Polysorbat 80 dazugegeben. Nach erfolgter Sterilfiltration wird mikrobiologisch reine Hydroxyäthylcellulose eingestreut und die Lösung unter Rühren 20 Min. auf 80° C erwärmt. Nach dem Abkühlen auf RT wird unter vorsichtigem Rühren die salzsaure, polysorbathaltige IFN-alpha-2-Lösung, bestehend aus einem in 0,01N HCl und 0,1 % Polysorbat 80 gelöstem IFN-alpha-2-Lyophilisat, dazugegeben und homogen verteilt. Die Abfüllung dieses Gemisches erfolgt unter laminaren Air Flow-Bedingungen in sterilen Aluminiumtuben.

Beispiel 3 (Hydrogel)

In 100 g Gel-Substanz sind enthalten :

| | |
|---|---|
| - IFN-alpha-2 | $1 \times 10^6$ I. E. |
| - Hydroxyäthylcellulose (z. B. Natrosol$^R$ 250 HX) | 1, 0 g |
| - Gelatine, Bloom 160 - 200 | 1, 0 g |
| - Polysorbat 20 | 0, 1 g |
| - Glycerin | 5, 0 g |
| - Bernsteinsäure | 0, 24 g |
| - Chlorhexidindigluconat | 0, 05 g |
| - 1N-NaOH (ad pH 6, 0) | |
| - Wasser, deion. | ad 100, 0 g |

Die Herstellung des IFN-alpha-2-Hydrogels erfolgt dadurch, daß Chlorhexidindigluconat, Glycerin und Gelatine bei ca. 30° C in Lösung gebracht werden. Anschließend wird Bernsteinsäure zugesetzt, mit 1N NaOH auf pH 6,0 eingestellt und Polysorbat 20 zugegeben.

6

Dann wird mikrobiologisch reine Hydroxyäthylcellulose eingestreut und die Lösung unter Rühren 20 Min. auf 80° C erwärmt. Nach dem Abkühlen auf RT wird unter vorsichtigem Rühren die salzsaure, polysorbathaltige IFN-alpha-2-Lösung dazugegeben und homogen verteilt. Die Abfüllung erfolgt wie bei Beispiel 1 und 2.

Beispiel 4 (Hydrogel als Nasengel)

In 100 g Gel-Substanz sind enthalten :

| | |
|---|---|
| - IFN-alpha-2 | $1 \times 10^8$ I. E. |
| - Hydroxypropylmethylcellulose (z. B. Methocel$^R$ Premium) | 0,1 g |
| - Polysorbat 60 | 0,1 g |
| - Gelatine, Bloom 160 - 200 | 0,5 g |
| - Sorbitlösung 70 % | 2,0 g |
| - Citronensäure x $H_2O$ | 0,130 g |
| - $Na_2HPO_4$ x 2 $H_2O$ | 0,30 g |
| - NaCl | 0,5 g |
| - Benzalkoniumchlorid 90 % | 0,02 g |
| - Wasser deion. | ad 100 g |

Die Herstellung des IFN-alpha-2-Hydrogels erfolgt dadurch, daß in 40° C warmem Wasser die Hydroxypropylmethylcellulose unter Rühren gelöst wird. Anschließend wird auf RT abgekühlt, während der Abkühlphase wird Gelatine in die Lösung eingebracht. Nach erfolgter Lösung werden nacheinander eingetragen: Benzalkoniumchlorid, Sorbitlösung, Zitronensäure, Di-Na-Hydrogenphosphat, NaCl und Polysorbat 60. In das fertige Gel wird bei RT unter vorsichtigem Rühren die salzsaure, 1 % Polysorbat-haltige IFN-alpha-2-Lösung eingebracht und homogen verteilt. Die Abfüllung erfolgt unter Laminar-Air-Flow-Bedingungen in sterile Aluminium-Tuben.

Beispiel 5 (Ovula)

In 100 g Ovula-Masse sind enthalten:

| | | |
|---|---|---|
| -IFN-alpha-2 | $1 \times 10^8$ I.E. | |
| -Hydroxyäthylcellulose | 0,1 | g |
| -Gelatine, Bloom 160 -200 | 10,0 | g |
| -$Na_2HPO_4$ x $2H_2O$ | 0,16 | g |
| -$KH_2PO_4$ | 0,09 | g |
| -Polysorbat 20 | 0,1 | g |
| -Glycerin | 57,0 | g |
| -Wasser deion. | ad 100 | g |

Die Herstellung der IFN-alpha-2-Ovula erfolgt dadurch, daß die Hydroxyethylcellulose zusammen mit der Gelatine im Glycerin-Wassergemisch, welches schon $Na_2HPO_4$ x $2H_2O$, $KH_2PO_4$ sowie Polysorbat enthält bei ca. 40-50° C gelöst wird. Im noch viskosen Zustand (bei ca. 37-40° C) wird die 1 % Polysorbathaltige, salzsaure (0,001N) IFN-alpha-2-Lösung vorsichtig eingerührt und die Lösung sofort zur Erstarrung in geeignete, gekühlte Formen ausgegossen.

Beispiel 6 (Stabilitätstests, Hydrogel ohne Gelatine-vs. mit Gelatine-Zusatz)

In 100 g Gelsubstanz sind enthalten:

| | | | | |
|---|---|---|---|---|
| -IFN-alpha-2 | $1 \times 10^8$ I.E. | | $1 \times 10^8$ I.E. | |
| -Nipagin M | 0,2 | g | 0,2 | g |
| -Gelatine, Bloom 160 -200 | - | | 0,5 | g |
| -NaCl | 0,48 | g | 0,48 | g |
| -KCl | 0,012 | g | 0,012 | g |
| -$Na_2HPO_4$ x $2H_2O$ | 0,086 | g | 0,086 | g |
| -$KH_2PO_4$ x $3H_2O$ | 0,012 | g | 0,012 | g |
| -Polysorbat 20 | 0,1 | g | 0,1 | g |
| -Hydroxyäthylcellulose | 2,0 | g | 2,0 | g |
| -(z. B. Natrosol$^R$ 250 HX) | | | | |
| -Wasser deion. | ad 100 | g | | |

Die punktierten Kurven in Abb.1 bis 3 beschreiben den Stabilitätsverlauf eines IFN-alpha-2-Hydrogeles obiger Zusammensetzung ohne Gelatine-Zusatz, die durchgezogenen Kurven mit Gelatine-Zusatz abgefüllt in Aluminium-Tuben. Jeder Punkt stellt den Mittelwert aus 6 Bestimmungen dar. Die Bestimmungen wurden mit dem ELISA-Test durchgeführt.

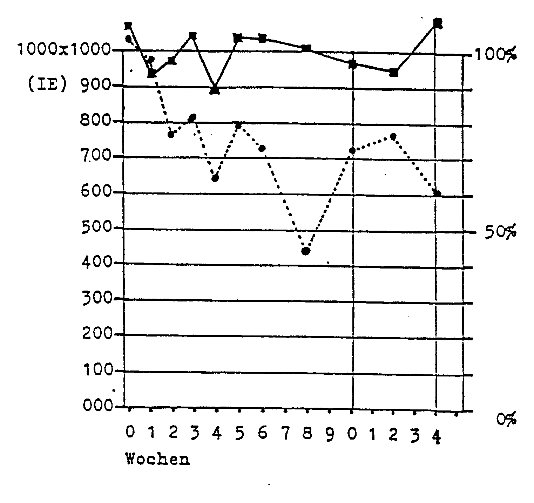

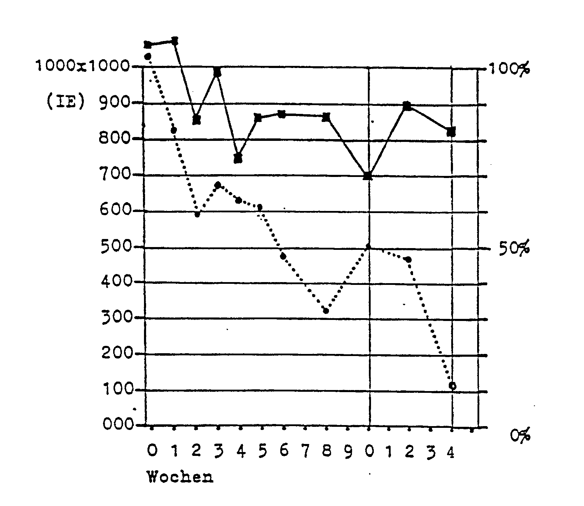

In Abbildung 1 ist der Stabilitätsverlauf bei Minus 10° C Lagerung, in Abbildung 2 bei Plus 4° C Lagerung und in Abbildung 3 bei Plus 21° C Lagerung, jeweils vergleichend, wiedergegeben.

Das Stabilitätsverhalten wurde über einen Zeitraum von 14 Wochen (= 3 1/2 Monate) verfolgt. Dabei konnte erstaunlicherweise und völlig unerwartet festgestellt werden, daß mit zunehmender Lagertemperatur die Stabilitätsunterschiede deutlicher wurden. Bei Summation der biologischen Stabilitätsergebnisse über den gesamten Zeitraum von 14 Wochen und Bildung des arithmetischen Mittels (x) der Werte (n = 11) ergibt sich das in der folgenden Tabelle dargestellte Bild, wobei ein Wert von 10,0 entsprechend $10^8$ I.E. IFN-alpha-2 pro 100 g, als 100% Aktivität gesetzt wurde:

8

| Lager-temperatur | Hydrogel ohne Gelatinezusatz | Hydrogel mit Gelatinezusatz | Dif-ferenz |
|---|---|---|---|
| -10°C | $\bar{x}$ (11) =8,49 (= -15%) | $\bar{x}$ (11) = 9.72 (= -3%) | 12% |
| + 4°C | $\bar{x}$ (11) =7.52 (= -25%) | $\bar{x}$ (11) = 9.97 (=+/-0) | 25% |
| +21°C | $\bar{x}$ (11) =5.67 (= -43%) | $\bar{x}$ (11) = 8.87 (=-11%) | 32% |

Aus dieser Tabelle kann man deutlich die stabilisierende Wirkung der Kombination von Hydroxyethylcellulose und Gelatine ablesen. Bei einer Lagerung im Kühlschrank (= plus 4°C) konnte praktisch keine Abnahme der Aktivität nach 3 1/2 Monaten der Formulierung mit Gelatinezusatz festgestellt werden, während die Formulierung ohne Gelatinezusatz einen Abfall von ca. 25 % zeigte. Kühlschranktemperatur stellt dabei eine praktikable Lagerung für ein Handelspräparat dar. Bei einer Lagerung bei 21°C war ein Aktivitätsverlust der Formulierung ohne Gelatinezusatz innerhalb des Lagerzeitraumes von 3 1/2 Monaten auf fast die Hälfte des Ausgangswertes festzustellen, während die erfindungsgemäße Formulierung mit Hydroxyäthylcellulose und Gelatine innerhalb dieses Zeitraumes nur einen Aktivitätsverlust von 11% aufwies, was einer relativen Stabilitätserhöhung von IFN alpha 2 von über 30% gleichkommt.

Ein weiterer Lagerungsversuch über 44 Wochen (10 Monate) einer Zubereitungsform nach Beispiel 1, abgefüllt in Aluminiumtuben mit Innenschutzlack, zeigte im Elisatest bei Lagertemperaturen von 4°C und 21°C keinen Aktivitätsabfall gegenüber dem Anfangswert.

| Lagertemperatur | Hydrogel gemäß Beispiel 1 | |
|---|---|---|
| | Anfangswert | Endwert nach 10 Monaten |
| + 4°C | $\bar{x}$ (6) 9,6 | $\bar{x}$ (6) 11,0 |
| + 21°C | $\bar{x}$ (6) 9,6 | $\bar{x}$ (6) 9,6 |

## Ansprüche

1.  Pharmazeutische Zubereitungsformen für die topische Anwendung enthaltend Interferon alpha, dadurch gekennzeichnet, daß diese als Träger und Stabilisator für das Interferon alpha

    1. Hydroxyethylcellulose, Hydroxypropylmethylcellulose oder Mischungen davon und
    2. Gelatine, sauer oder alkalisch aufgeschlossen, sowie zusätzlich als Antiadhäsionsmittel anionaktive, kationaktive oder neutrale oberflächenaktive Substanzen und, gegebenenfalls, weitere übliche Hilfsstoffe enthalten.

2.  Pharmazeutische Zubereitungsformen für die topische Anwendung enthaltend Interferon alpha nach Anspruch 1, dadurch gekennzeichnet, daß diese ein Hydrogel sind.

3.  Pharmazeutische Zubereitungsformen nach Anspruch 2, dadurch gekennzeichnet, daß diese als Träger und Stabilisator Hydroxyethylcellulose und Gelatine enthalten, wobei die auf das fertige Produkt bezogenen Menge an Hydroxyethylcellulose von 0,1 bis 2,0 Gew.-% beträgt.

4. Pharmazeutische Zubereitungsformen nach Anspruch 2, dadurch gekennzeichnet, daß diese als Träger und Stabilisator Hydroxypropylmethylcellulose und Gelatine enthalten, jeweils in einer auf das fertige Produkt bezogenen Menge von 0,1 bis 2,0 Gew.-%.

5. Pharmazeutische Zubereitungsformen nach Anspruch 2, dadurch gekennzeichnet, daß diese als Träger und Stabilisator Hydroxyäthylcellulose und Gelatine enthalten, wobei die auf das fertige Produkt bezogene Menge an Gelatine von 0,1 bis 10 Gew.-% beträgt.

6. Pharmazeutische Zubereitungsformen nach einem der Ansprüche 1 bis 5, dadurch gekennzeichnet, daß das Antiadhäsionsmittel aus polyäthoxylierten Sorbitan-Estern besteht.

7. Pharmazeutische Zubereitungsformen nach einem der Ansprüche 1 bis 6, dadurch gekennzeichnet, daß das darin enthaltene Interferon alpha aus einer Zellkultur erhalten wird, die mit einer für Human Interferon alpha codierenden DNA-Sequenz transformiert wurde.

8. Pharmazeutische Zubereitungsformen nach einem der Ansprüche 1 bis 7, dadurch gekennzeichnet, daß diese als zusätzliche Hilfsstoffe ein Puffersystem für einen pH-Bereich von 6,0 bis 7,4, Feuchthaltesubstanzen, hautverträgliche Konservierungsstoffe, Interferon alpha - kompatible Penetrationsförderer und ein Vehikel enthalten.

9. Pharmazeutische Zubereitungsformen nach Anspruch 8, dadurch gekennzeichnet, daß es als Feuchthaltesubstanzen Glycerin, 1,2-Propylenglykol, Sorbit, Butylenglykol, ein Polyol wie Polyethylenglykol 400 oder ein Gemisch davon enthält.

10. Pharmazeutische Zubereitungsformen nach Anspruch 8, dadurch gekennzeichnet, daß der für Interferon alpha kompatible Penetrationsförderer bis zu 50 Gew.-% Dimethylsulfoxid ist.

11. Verfahren zur Herstellung von pharmazeutischen Zubereitungsformen gemäß Anspruch 1 für die topische Anwendung mit Stabilisierung des darin enthaltenen Interferon alpha, dadurch gekennzeichnet, daß zur Herstellung eines Hydrogels Gelatine, in einer auf das fertige Produkt bezogenen Menge von 0,1 bis 2,0 Gew.-%, bei Temperaturen bis zu 80 °C in Wasser gelöst wird, die übrigen Hilfsstoffe, wie Feuchthalter, Salze, Puffersysteme und Penetrationsförderer, anschließend in die Lösung eingebracht werden, hernach Hydroxyethylcellulose, Hydroxypropylmethylcellulose oder ein Gemisch davon, in die Lösung eingebracht wird und in dieser Lösung nach dem Abkühlen auf Raumtemperatur Interferon alpha in einer Menge von $1 \times 10^5$ bis $1 \times 10^{10}$ IE/100 g fertiges Produkt homogen verteilt und dieses Gemisch steril abgefüllt wird.

12. Verfahren zur Herstellung von pharmazeutischen Zubereitungsformen gemäß Anspruch 1 für die intravaginale Applikation, dadurch gekennzeichnet, daß
a. Hydroxyethylcellulose in einer Menge von 0,1 bis 2,0 Gew.-% zusammen mit Gelatine in einer, Menge bis zu 10 Gew.-% bei Temperaturen bis zu 60 °C in einem Wasser-Glycerin-Gemisch, welches ein Antiadhäsionsmittel, und gegebenenfalls Salze, Puffersubstanzen und Konservierungsmittel enthält, gelöst werden, und
b. bei einer Temperatur von 40 bis 50 °C eine Polysorbat-haltige salzsaure IFN alpha-Lösung vorsichtig eingerührt und
c. diese Lösung sofort in geeignete Formen ausgegossen wird.

Patentansprüche für folgende Vertragsstaaten : AT, GR, ES

1. Verfahren zur Herstellung pharmazeutischer Zubereitungsformen für die topische Anwendung enthaltend Interferon alpha, als Träger und Stabilisator für das Interferon alpha Hydroxyethylcellulose, Hydroxypropylmethylcellulose oder Mischungen davon und Gelatine, sauer oder alkalisch aufgeschlossen, desweiteren, anionaktive kationaktive oder neutrale oberflächenaktive Substanzen als Antiadhäsionsmittel und, gegebenenfalls, weitere Hilfsstoffe, dadurch gekennzeichnet, daß zur Herstellung eines Hydrogels Gelatine, in einer auf das fertige Produkt bezogenen Menge von 0,1 bis 2,0 Gew.-%, bei Temperaturen bis zu 80°C in Wasser gelöst wird, die übrigen Hilfsstoffe, wie Feuchthalter, Salze, Puffersysteme und Penetrationsförderer, anschließend in die Lösung eingebracht werden, hernach Hydroxyethylcellulose, Hydroxypropylmethylcellulose oder ein Gemisch davon in die Lösung eingebracht wird und in dieser Lösung nach dem Abkühlen auf Raumtemperatur Interferon alpha in einer Menge von 1 x10$^5$ bis 1 x 10$^{10}$ IE/100 g fertiges Produkt homogen verteilt und dieses Gemisch steril abgefüllt wird.

2. Verfahren zur Herstellung von pharmazeutischen Zubereitungsformen gemäß Anspruch 1 für die intravaginale Applikation, dadurch gekennzeichnet, daß

   a. Hydroxyethylcellulose in einer Menge von 0,1 bis 2,0 Gew.-% zusammen mit Gelatine in einer Menge bis zu 10 Gew.-% bei Temperaturen bis zu 60°C in einem Wasser-Glycerin-Gemisch, welches gegebenenfalls noch ein Antiadhäsionsmittel, Salze, Puffersubstanzen und Konservierungsstoffe enthält, gelöst werden, und

   b. bei einer Temperatur von 40 bis 50°C eine Polysorbat-haltige salzsaure IFN alpha-Lösung vorsichtig eingerührt und

   c. diese Lösung sofort in geeignete Formen ausgegossen wird.

3. Verfahren gemäß Anspruch 1 und 2, dadurch gekennzeichnet, daß desweiteren anorganische oder organische Puffer für einen pH-Bereich von 6,0 bis 7,4, Feuchtehaltesubstanzen und gegebenenfalls Konservierungsstoffe und Penetrationsförderer eingearbeitet werden.

4. Verfahren gemäß Anspruch 3, dadurch gekennzeichnet, daß als oberflächenaktive Substanzen polyäthoxylierte Sorbitan-Ester, als Puffer Natriumphosphat-Puffer, Kaliumhydrogenphosphat-Natriumdihydrogenphosphat-Puffer, Zitronensäurephosphat-Puffer, Kalium-Natrium-glutamat-phosphat-Puffer und Succinat-Puffer, als Feuchthaltesubstanzen Glycerin, 1,2-Propylenglykol, Sorbit, Butylenglykol, Polyole, gegebenenfalls, als Penetrationsförderer Dimethylsulfoxid, und als Vehikel Wasser, sofern nichtalkoholische Hydrogele vorliegen, eingearbeitet werden.

## Claims

1. Pharmaceutical formulations for topical administration containing α-interferon, characterised in that they contain as excipient and stabiliser for the α-interferon

   1. hydroxyethyl cellulose, hydroxypropylmethyl cellulose or mixtures thereof, and

   2. gelatine, which has been subjected to acid or alkali digestion, and, in addition, as anti-adhesion agent, anionic, cationic or neutral surface-active substances and, where appropriate, other conventional adjuvants.

2. Pharmaceutical formulations for topical administration containing α-interferon according to claim 1, characterised in that they comprise a hydrogel.

3. Pharmaceutical formulations according to claim 2, characterised in that they contain as excipient and stabiliser hydroxyethyl cellulose and gelatine, the amount of hydroxyethyl cellulose being from 0.1 to 2.0% by weight, based on the finished product.

4. Pharmaceutical formulations according to claim 2, characterised in that they contain as excipient and stabiliser hydroxypropylmethyl cellulose and gelatine, each being in an amount of 0.1 to 2.0% by weight, based on the finished product.

5. Pharmaceutical formulations according to claim 2, characterised in that they contain as excipient and stabiliser hydroxyethyl cellulose and gelatine, the amount of gelatine being 0.1 to 10% by weight, based on the finished product.

6. Pharmaceutical formulations according to one of claims 1 to 5, characterised in that the anti-adhesion agent is composed of polyethoxylated sorbitan esters.

7. Pharmaceutical formulations according to one of claims 1 to 6, characterised in that the α-interferon contained therein is obtained from a cell culture which has been transformed with a DNA sequence coding for human α-interferon.

8. Pharmaceutical formulations according to one of claims 1 to 7, characterised in that they contain as additional adjuvants a buffer system for a pH range from 6.0 to 7.4, humectants, preservatives which are tolerated by skin, penetration promoters which are compatible with α-interferon and a vehicle.

9. Pharmaceutical formulations according to claim 8, characterised in that they contain, as humectants, glycerol, 1,2-propylene glycol, sorbitol, butylene glycol, a polyol such as polyethylene glycol 400 or a mixture thereof.

10. Pharmaceutical formulations according to claim 8, characterised in that the penetration promoter which is compatible with α-interferon is up to 50% by weight of dimethyl sulphoxide.

11. Process for the preparation of pharmaceutical formulations according to claim 1 for topical use which stabilise the α-interferon contained therein, characterised in that, in order to prepare a hydrogel, gelatine in an amount of 0.1 to 2.0% by weight, based on the finished product, is dissolved in water at temperatures up to 80° C, and the other adjuvants such as humectants, salts, buffer systems and penetration-promoters are then introduced into this solution, then hydroxyethyl cellulose, hydroxypropylmethyl cellulose or a mixture thereof is introduced into the solution and, after cooling to ambient temperature, α-interferon, in an amount of $1 \times 10^5$ to $1 \times 10^{10}$ IV/100 g of finished product is homogeneously dispersed therein, and this mixture is filled into sterile containers.

12. Process for the preparation of pharmaceutical formulations according to claim 1 for intra-vaginal administration, characterised in that
    a. hydroxyethyl cellulose in an amount of 0.1 to 2.0% by weight is dissolved together with gelatine in an amount of up to 10% by weight, at temperatures up to 60° C in a water/glycerol mixture which also contains an anti-adhesion agent and optionally salts, buffer substances and preservatives, and
    b. at a temperature of 40 to 50° C, an α-IFN solution which contains polysorbate and hydrochloric acid is cautiously stirred in, and
    c. this solution is immediately poured out into suitable moulds.

Claims for the following Contracting States : AT, GR, ES

1. Process for preparing pharmaceutical formulations for topical use containing α-interferon, and, as excipient and stabiliser for the α-interferon, hydroxyethyl cellulose, hydroxypropylmethyl cellulose or mixtures thereof and gelatine, which has been subjected to acid or alkaline digestion, and furthermore anionic, cationic or neutral surface-active substances as antiadhesion agents, and optionally other adjuvants, characterised in that, in order to prepare a hydrogel, gelatine in an amount of 0.1 to 2.0% by weight, based on the finished product, is dissolved in water at temperatures up to 80° C, and the other adjuvants such as humectants, salts, buffer systems and penetration-promoters are then introduced into this solution, then hydroxyethyl cellulose, hydroxypropylmethyl cellulose or a mixture thereof is introduced into the solution and, after cooling to ambient temperature, α-interferon, in an amount of $1 \times 10^5$ to $1 \times 10^{10}$ IU/100 g of finished product is homogeneously dispersed therein, and this mixture is filled into sterile containers.

2. Process for the preparation of pharmaceutical formulations according to claim 1 for intra-vaginal administration, characterised in that
    a. hydroxyethyl cellulose in an amount of 0.1 to 2.0% by weight is dissolved together with gelatine in an amount of up to 10% by weight, at temperatures up to 60° C in a water/glycerol mixture which also contains an anti-adhesion agent and optionally salts, buffer substances and preservatives, and
    b. at a temperature of 40 to 50° C, an α-IFN solution which contains polysorbate and hydrochloric acid is cautiously stirred in, and
    c. this solution is immediately poured out into suitable moulds.

3. Process according to claims 1 and 2, characterised in that inorganic or organic buffers for a pH range of 6.0 to 7.4, humectants and optionally preservatives and penetration promoters are also incorporated.

4. Process according to claim 3, characterised in that the surface-active substances incorporated are polyethoxylated sorbitan esters, the buffers are sodium phosphate buffer, potassium hydrogen phosphate-sodium dihydrogen phosphate buffer, citric acid phosphate buffer, potassium-sodium glutamate phosphate buffer and succinate buffer, the humectant substances are glycerol, 1,2-propyleneglycol, sorbitol, butyleneglycol, polyols, and optionally, as penetration promoters, dimethyl sulphoxide, and as the vehicle, water, if non-alcoholic hydrogels are present.

## Revendications

1. Formes de préparations pharmaceutiques pour l'utilisation topique contenant de l'interféron alpha, caractérisées en ce qu'elles contiennent comme support et stabilisant pour l'interféron alpha
   1. de l'hydroxyéthylcellulose, de l'hydroxypropylméthylcellulose ou des mélanges de celles-ci et
   2. de la gélatine, désagrégée par voie acide ou alcaline,
      ainsi qu'en outre des substances tensioactives à activité anionique, à activité cationique ou neutres comme agents antiadhésion et, éventuellement, d'autres adjuvants usuels.

2. Formes de préparations pharmaceutiques pour l'utilisation topique contenant de l'interferon alpha selon la revendication 1, caractérisées en ce qu'elles sont un hydrogel.

3. Formes de préparations pharmaceutiques selon la revendication 2, caractérisées en ce qu'elles contiennent comme support et stabilisant de l'hydroxyéthylcellulose et de la gélatine, la quantité d'hydroxyéthylcellulose par rapport au produit fini étant de 0,1 à 2,0 % en poids.

4. Formes de préparations pharmaceutiques selon la revendication 2, caractérisées en ce qu'elles contiennent comme support et stabilisant de l'hydroxypropylméthylcellulose et de la gélatine, chaque fois en une quantité de 0,1 à 2,0 % en poids par rapport au produit fini.

5. Formes de préparations pharmaceutiques selon la revendication 2, caractérisées en ce qu'elles contiennent comme support et stabilisant de l'hydroxyéthylcellulose et de la gelatine, la quantité de gélatine par rapport au produit fini étant de 0,1 à 10% en poids.

6. Formes de préparations pharmaceutiques selon l'une des revendications 1 à 5, caractérisées en ce que l'agent antiadhésion est constitué d'esters de sorbitane polyéthoxylés.

7. Formes de préparations pharmaceutiques selon l'une des revendications 1 à 6, caractérisées en ce que l'interféron alpha qui y est contenu est obtenu à partir d'une culture cellulaire qui a été transformée par une séquence ADN codant pour l'interféron alpha humain.

8. Formes de préparations pharmaceutiques selon l'une des revendication 1 à 7, caractérisées en ce qu'elles contiennent comme adjuvant supplémentaire un système tampon pour un domaine de pH de 6,0 à 7,4, des substances de maintien de l'humidité, des agents de conservation compatibles avec la peau, des agents facilitant la pénétration, compatibles avec l'interféron alpha et un véhicule.

9. Formes de préparations pharmaceutiques selon la revendication 8, caractérisées en ce qu'elles contiennent comme subtances de maintien de l'humidité, du glycérol, du l,2-propylèneglycol, du sorbitol, du butylèneglycol, un polyol tel que le polyéthylèneglycol 400 ou un mélange de ceux-ci.

10. Formes de préparations pharmaceutiques selon la revendication 8, caractérisées en ce que l'agent favorisant la pénétration compatible pour l'interféron alpha est constitué jusqu'à 50% en poids de diméthylsulfoxyde.

11. Procédé pour la fabrication de formes de préparations pharmaceutiques selon la revendication 1, pour l'utilisation topique avec stabilisation de l'interféron alpha qui y est contenu, caractérisé en ce que
    pour la fabrication d'un hydrogel
    de la gélatine est dissoute dans l'eau à des températures allant jusqu'à 80°C, en une quantité de 0,1 jusqu'à 2,0 % en poids par rapport au produit fini, les autres adjuvants tels qu'agent de maintien de l'humidité, sels, systèmes tampons et agents favorisant la pénétration sont ensuite introduits dans la solution, à la suite de quoi de l'hydroxyéthylcellulose, de l'hydroxypropylméthylcellulose ou un

13

mélange de celles-ci est introduit dans la solution et en ce que dans cette solution, après refroidissement à la température ambiante, de l'interféron alpha est réparti de façon homogène en une quantité de 1 x 10⁵ jusqu'à 1 x 10¹⁰ UI/100 g de produit fini et en ce que ce mélange est conditionné de façon stérile.

12. Procédé pour la fabrication de formes de préparations pharmaceutiques selon la revendication 1 pour l'application intravaginale, caractérisé en ce que

a. de l'hydroxyéthylcellulose en une quantité de 0,1 à 2,0 % en poids, ensemble avec de la gélatine en une quantité allant jusqu'à 10 % en poids, sont dissoutes à des températures allant jusqu'à 60° C dans un mélange eau-glycérol qui contient un agent antiadhésion, et éventuellement des sels, des substances tampons et des agents de conservation, et

b. à un température de 40 à 50° C une solution chlorhydrique d'IFN alpha contenant du polysorbate est introduite avec précaution, en agitant et

c. cette solution est immédiatement versée dans des moules appropriés.

Revendications pour les Etats contractants suivants: AT, GR, ES

1. Procédé pour la fabrication de formes de préparations pharmaceutiques pour l'utilisation topique, contenant de l'interféron alpha, comme support et stabilisant pour l'interféron alpha de l'hydroxyéthylcellulose, de l'hydroxypropylméthylcellulose ou des mélanges de celles-ci et de la gélatine, désagrégée par voie acide ou alcaline, en outre des substances tensioactives à activité anionique, à activité cationique ou neutres en tant qu'agents antiadhésion et, éventuellement, d'autres adjuvants, caractérisé en ce que

pour la fabrication d'un hydrogel

de la gélatine est dissoute dans l'eau à des températures allant jusqu'à 80° C en une quantité de 0,1 à 2,0 % en poids par rapport au produit fini, les autres adjuvants tels qu'agents de maintien de l'humidité, sels, systèmes tampons et agents favorisant la pénétration sont ensuite introduits dans la solution, à la suite de quoi l'hydroxyéthylcellulose, l'hydroxypropylméthylcellulose ou un mélange de celles-ci est introduit dans la solution et, après refroidissement à la température ambiante, de l'interféron alpha est réparti de façon homogène dans cette solution en une quantité de 1 x 10⁵ jusqu'à 1 x 10¹⁰ UI/100 g de produit fini et ce mélange est conditionné de façon stérile.

2. Procédé pour la fabrication de formes de préparations pharmaceutiques selon la revendication 1 pour l'application intravaginale, caractérisé en ce que

a. de l'hydroxyéthylcellulose en une quantité de 0,1 à 2,0 % en poids, ensemble avec de la gélatine en une quantité allant jusqu'à 10 % en poids, sont dissoutes à des températures allant jusqu'à 60° C dans un mélange eau-glycérol qui contient éventuellement aussi un agent antiadhésion, des sels, des substances tampons et des agents de conservation et

b. une solution chlorhydrique d'IFN alpha contenant du polysorbate est introduite avec précaution, en agitant, à une température de 40 à 50°C et

c. cette solution est immédiatement versée dans des moules appropriés.

3. Procédé selon la revendication 1 et 2, caractérisé en ce qu'en outre des tampons non organiques ou organiques pour un domaine de pH de 6,0 à 7,4, des substances de maintien de l'humidité et éventuellement des agents de conservation et des agents favorisant la pénétration sont introduits.

4. Procédé selon la revendication 3, caractérisé en ce qu'on introduit comme subtances tensioactives des esters de sorbitane polyéthoxylés, comme tampons du tampon phosphate de sodium, du tampon phosphate acide de potassium-phosphate diacide de sodium, du tampon acide citrique-phosphate, du tampon glutamate-phosphate de potassium et de sodium et du tampon succinate, comme substances maintenant l'humidité du glycérol, du 1,2-propylèneglycol, du sorbitol, du butylèneglycol, des polyols, éventuellement comme agent favorisant la pénétration du diméthylsulfoxyde et comme véhicule de l'eau, dans la mesure où on est en présence d'hydrogels non alcooliques.